# NEUE EUROPÄISCHE PATENTSCHRIFT

(11) **EP 2 497 451 B2**
(45) Veröffentlichungstag und Bekanntmachung des Hinweises auf die Entscheidung über den Einspruch: **07.05.2025**
(45) Hinweis auf die Patenterteilung: 18.06.2014
(21) Anmeldenummer: 12001502.9
(22) Anmeldetag: 06.03.2012
(51) Int. Cl.: A61F 13/15, A61F 13/47, A61F 13/532

(54) **Bindenprodukt, Verfahren zum Herstellen eines Hygieneproduktes und Vorrichtung zum Herstellen eines Hygieneproduktes**
Hygiene product, method for producing a hygiene product and device for producing a hygiene product
Produit hygiènique, procédé de fabrication d'un produit d'hygiène et dispositif de fabrication d'un produit d'hygiène

(30) Priorität: 11.03.2011 DE 102011013707
(43) Veröffentlichungstag der Anmeldung: 12.09.2012
(73) Patentinhaber: W. Pelz GmbH & Co. KG, 23812 Wahlstedt/Holstein (DE)
(72) Erfinder: Bark, Andreas, 22397 Hamburg (DE)
(74) Vertreter: Hauck Patentanwaltspartnerschaft mbB

(56) Entgegenhaltungen:
- EP-A1- 1 621 165
- DE-A1- 19 806 575
- US-A- 4 960 477
- US-A- 4 994 053
- US-A- 5 494 622
- US-A- 5 593 399
- US-A- 5 925 439
- US-A1- 2003 118 780
- US-A1- 2008 044 616
- US-A1- 2010 022 978

## Beschreibung

Die Erfindung bezieht sich auf eine Vorrichtung zum Herstellen eines Hygieneproduktes.

Binden- bzw. Hygieneprodukte dienen dazu, Flüssigkeiten wie Menstruationsflüssigkeit, Sekret oder Urin aufzunehmen. Hierbei handelt es sich insbesondere um Slipeinlagen, Damenbinden, Monatsbinden und Inkontinenzbinden, die in dieser Anmeldung als "Bindenprodukt" oder "Binden" bezeichnet werden. Hygieneprodukte können Bindenprodukte, Babywindeln und andere Flüssigkeit aufnehmende Produkte für die Körperhygiene sein.

Bei derartigen Hygieneartikeln kommen Superabsorber, kurz "SAP" zum Einsatz. Hierbei handelt es sich um spezielle Materialien, die sich durch eine enorme Wasseraufnahmefähigkeit auszeichnet. Chemisch kann es sich bei einem Superabsorber um ein Copolymer aus Acrylsäure und Natriumacrylat handeln. SAP kommen meist als grobkörniges Pulver zum Einsatz. Sie werden in einen Träger integriert, der als Kern eines Hygieneproduktes verwendet wird, der die vom Hygieneprodukt aufgenommene Flüssigkeit speichert.

Bereits bekannt sind Hygieneprodukte mit einem Kern aus Cellulose und Superabsorber, der mit Hilfe eines Flockenlegers hergestellt wird. Der Kern ist meist mehrschichtig aufgebaut und hierdurch ist es möglich, den Superabsorber in bestimmten Schichten des Kerns in höherer Konzentration einzuarbeiten. Zu diesen Produkten zählen zumeist Inkontinenzartikel und Babywindeln.

Ferner bekannt sind Hygieneprodukte, die ein Vlies aus vorgefertigter Zellulose enthalten, das im Allgemeinen als "Airlaid" bezeichnet wird. Das Airlaid kann von vornherein einen Superabsorber enthalten. Bei der Herstellung des Hygieneproduktes kann aber auch ein ursprünglich von SAP freies Airlaid zum Einsatz kommen. In das Airlaid wird bei der Herstellung des Hygieneproduktes SAP eingestreut. Dieser wird mit Heißklebstoff ("Hotmelt") fixiert. Danach wird das Airlaid gefaltet, sodass der SAP zwischen den einander berührenden Innenseiten des Airlaid gefangen ist. Slipeinlagen und besonders dünne Binden ("Ultrabinden") verwenden Airlaids als Kern. Darin ist der Superabsorber gleichmäßig verteilt.

Ein erheblicher Anteil der Materialkosten von Hygieneprodukten entfällt auf den eingesetzten Superabsorber.

US 2008/044616 A1 beschreibt Absorbtionsartikel so wie Papierwindeln und Binden. Die Ausführungsbcispiele betreffen Absorbtionsartikel, bei denen der Superabsorber im Zentralbereich des Kerns eine höhere Konzentration als in den Seitenbereichen des Kerns aufweist. Der Superabsorber ist im Zentralbereich und in den Seitenbereichen jeweils gleichmäßig verteilt.

DE 198 06 575 A1 beschreibt absorbierende Artikel mit einer flüssigkcitsdurchlässigen Schicht, einer flüssigkeitsundurchlässigen Schicht und einem Saugkörper dazwischen. Der Saugkörper weist ein Kernstück und eine flüssigkeitsdurchlässige Umhüllung auf. Das saugfähige Material des Kernstücks kann ein superabsorbierendes Material enthalten.

US 2010/022978 A1 beschreibt Absorbtionselementc mit Superabsorber, die in einem Zentralbereich eine höhere Konzentration als in Seitenbereichen aufweisen. Gemäß einer Ausgestaltung nimmt die Konzentration des Superabsorbers in Längsrichtung vom Zentralbereich auch zu den beiden Seitenbereichen hin allmählich ab.

US 2003/118780 A1 beschreibt die Herstellung von Stoffbahnen mit verschiedener Faserdichte durch Anlegen von Vakuum an einem Drahtsieb, auf dem die Fasern abgelagert werden. Dort, wo ein höheres Vakuum herrscht, lagern sich mehr Fasern ab. Zwischen dem Drahtsieb und der Vakuumquelle wird eine Lochplatte angeordnet, um den Unterdruck zu steuern. Die Verteilung des Superabsorbers in Maschinenlaufrichtung in zwei Zonen hoher Dichte und einer Zone geringerer Dichte erfolgt durch Steuerung eines Aufgabemittels für Superabsorber, unter dem die Stoffbahn hergeführt wird.

US 4,994,053A beschreibt ein Verfahren zum Herstellern eines zusammengesetzten Erzeugnisses aus einer flüssigkeitsundurchlässigen Stoffbahn und darauf in diskreten Bereichen abgelegtem Superabsorber, wobei zum Ablegen der diskreten Bereiche auf der Stoffbahn eine Walze mit Hohlräumen eingesetzt wird.

Zudem beschreibt US 2010/022978 A1 das Verteilen von Superabsorber auf einer Materialbahn in örtlich unterschiedlicher Konzentration. Hierfür wird der Superabsorber intermittierend aufgebracht oder wird die aufzubringende Menge beim Durchlauf der Materialbahn unter den Aufbringungsmitteln fortschreitet verändert, z.B. gesteigert und verringert.

Davon ausgehend liegt der Erfindung die Aufgabe zugrunde, eine Vorrichtung zum Herstellen eines Bindenproduktes und anderer Hygieneprodukte mit hinreichender Aufnahmefähigkeit für Flüssigkeit mit verringertem Materialaufwand zur Verfügung zu stellen.

Die Aufgabe wird durch ein Bindenprodukt mit den Merkmalen von Anspruch 1 gelöst. Vorteilhafte Ausgestaltungen des Bindenproduktes sind in Unteransprüchen angegeben.

Die nachfolgend beschriebenen Bindenprodukte sind nicht Gegenstand des Patents, sondern dienen nur dem besseren Verständnis der Erfindung.
Das Bindenprodukt hat
- einen streifenförmigen Kern aus einem Airlaid, Vlies oder einem anderen flexiblen Material aus natürlichen und/oder künstlichen Fasern und
- einen im Kern auf einer Vielzahl kleiner Flächen, die einen Abstand voneinander haben, verteilten Superabsorber, der in einem Zentralbereich des Kerns eine höhere Konzentration als in mindestens einem Seitenbereich des Kerns aufweist, der in Längsrichtung des Kerns außerhalb des Zentralbereiches angeordnet ist.

Die Erfindung besteht darin, den SAP über den Kern so zu verteilen, dass der SAP im Zentralbereich des Kerns eine höhere Konzentration aufweist, als in mindestens einem Seitenbereich des Kerns. Bevorzugt hat der SAP im Zentralbereich des Kerns eine höhere Konzentration als in beiden Seitenbereichen des Kerns, die beidseitig des Zentralbereiches angeordnet sind. Diese Ausgestaltung des Kerns eines Bindenproduktes hat den Vorteil, dass sich der SAP im Wesentlichen dort befindet, wo das Bindenprodukt die Körperflüssigkeit aufzunehmen hat.

Die Bindenprodukte werden nämlich von den Verwenderinnen so am Körper platziert, dass die Flüssigkeit mittig vom Kern aufgenommen werden kann. Von diesem zentralen Punkt der Beaufschlagung wird die Flüssigkeit im Kern mehr oder weniger radial nach außen verteilt Bedingt durch eine Faserstruktur des Kerns kann die Flüssigkeit in einer Vorzugsrichtung etwas schneller und weiter verteilt werden, als quer dazu, sodass sich eine leicht ovale Verteilung der Flüssigkeit im Kern ergeben kann. In der Regel sind die Fasern in Längsrichtung des Kerns ausgerichtet, da die Längsrichtung des Kerns der Maschinenlaufrichtung den Maschinen entspricht, auf denen das Bahnmaterial hergestellt wird, aus dem der Kern hergestellt wird. Infolgedessen kann sich die Flüssigkeit in Richtung der Längsachse des Kerns etwas schneller und weiter ausbreiten, als in Querrichtung des Kerns. Die Belastbarkeit des Produktes hinsichtlich der Gesamtflüssigkeitsaufnahme ist dadurch begrenzt, dass die Flüssigkeit an den Längsseiten des Kerns und/oder über die Oberfläche des Kerns austritt und die Wäsche der Verwenderin verschmutzt. Wenn diese Kapazitätsgrenze erreicht ist, bleiben bei herkömmlichen Hygieneprodukten erhebliche Kapazitätsreserven außerhalb des Verteilungsbereichs der Flüssigkeit ungenutzt. Dies gilt insbesondere für die in Längsrichtung beidseitig des Verteilungsbereichs der Flüssigkeit angeordneten Seitenbereiche des Kerns. Infolgedessen werden - grob geschätzt - nur etwa 50% des SAP eines herkömmlichen Hygieneproduktes mit gleichmäßig über den Kern verteiltem SAP für die Flüssigkeitsaufnahme genutzt.

Durch die höhere Konzentration des SAP im Zentralbereich als in einem oder mehreren Seitenbereichen des Kerns wird ein weitaus höherer Anteil des SAP in der gewünschten Weile genutzt. Dies ist bereits bei einem Kern gegeben, der im Zentralbereich eine höhere Konzentration als in nur einem der in Längsrichtung außerhalb des Kerns angeordneten Seitenbereichen hat. Bevorzugt weist der eingesetzte SAP im Zentralbereich des Kerns eine höhere Konzentration als in beiden Seitenbereichen auf, die in Längsrichtung außerhalb des Zentralbereiches angeordnet sind. Bei dieser Ausgestaltung können - grob geschätzt - etwa 90% des SAP für die Aufnahme von Flüssigkeit genutzt werden.

Das Bindenprodukt spart bei gleicher Leistungsfähigkeit wie bei einem herkömmlichen Bindenprodukt, bei dem der SAP gleichmäßig über den Kern verteilt ist, erhebliche Mengen an SAP ein. Die eingesetzte Menge SAP wird nämlich im Wesentlichen für die Aufnahme von Flüssigkeit ausgenutzt und reicht auch aus, die gewünschten Mengen an Flüssigkeit aufzunehmen. Umgekehrt kann ein Bindenprodukt mit signifikant gegenüber herkömmlichen Bindenprodukten erhöhtem Leistungsvermögen zur Verfügung gestellt werden, wenn dieselbe Menge SAP wie bei einem herkömmlichen Bindenprodukt eingesetzt wird.

Die Eigenschaften des Bindenproduktes werden durch Standard-Tests für Hygieneartikel bestätigt. Auch hier werden bestimmte Parameter, wie Einsickerzeit (auch "Aufnahmezeit" genannt) oder Rewet (auch "Rücknässung" genannt), ausschließlich im Zentrum des Hygieneproduktes bestimmt. Nur bei der Messung der Gesamtkapazität, bei der das Produkt in der vollen Länge mit Testflüssigkeit beaufschlagt wird, schneiden die erfindungsgemäßen Bindenprodukte mit im Zentrum konzentriertem SAP schlechter ab. Dieser Test aber ist nach den der Erfindung zugrunde liegenden Erkenntnissen praxisfern.

Bei dem Bindenprodukt ist besonders vorteilhaft, dass der Kern aus einem Airlaid oder aus einem ähnlich weichen und geschmeidigen, flüssigkeitsabsorbierenden Material besteht. Dies ermöglicht es, Binden herzustellen, die sich besonders gut an den Körper der Trägerin anschmiegen. Harte Stellen durch Materialanhäufungen auf dem Kern sowie Falten können vermieden werden. Vorzugsweise übersteigt hierfür die Schichtdicke des auf den Kern aufgetragenen Superabsorbers nicht 2mm. Weiterhin ist hierfür der Superabsorber auf einer Vielzahl kleiner Flächen, die voneinander einen Abstand haben, aufgetragen. Bevorzugt handelt es sich hierbei um eine Vielzahl kreisförmiger oder elliptischer oder mehreckiger Flächen. Darüber hinaus hat dies den Vorteil, dass es eine eigene Absorptionskapazität aufweist, die zur Absorptionsfähigkeit des SAP hinzukommt. Bevorzugt ist das Hygieneprodukt eine Slipeinlage, Dünnbinde oder Ultrabinde.

Gemäß einer Ausgestaltung hat der Superabsorber in einem Zentralbereich des Kerns eine höhere Konzentration als in mindestens einem weiteren Seitenbereich des Kerns, der in Querrichtung des Kerns außerhalb des Zentralbereichs angeordnet ist. Bevorzugt ist die Konzentration des Superabsorbers im Zentralbereich des Kerns höher als in beiden weiteren Seitenbereichen beidseitig des Zentralbereichs. Hierdurch wird der Tatsache Rechnung getragen, dass sich die Flüssigkeit auch in Querrichtung nicht überall über den Kern verteilt. Die Aufnahmefähigkeit des Kerns für Flüssigkeit ist zwar dadurch begrenzt, dass die Flüssigkeit in Querrichtung die Längsseiten des Kerns erreicht. Dies ist zuerst etwa in der Quermittelebene des Kerns der Fall. Dann hat die Flüssigkeit in einem Abstand von der Quermittelebene die Längsseiten des Kerns noch nicht erreicht. Der Abstand der Flüssigkeitsfront von den Längsseiten wächst mit dem Abstand von der Quermittelebene des Kerns. Dies ermöglicht es, den Superabsorber auch in Querrichtung des Kerns auf einen Zentralbereich zu konzentrieren.

Bevorzugt ist der Superabsorber in einem ovalen Zentralbereich des Kerns angeordnet. Dies entspricht der Ausbreitung der Flüssigkeit, die zentral auf den Kern aufgegeben wird. Der ovale Zentralbereich ist bevorzugt mit seiner Hauptachs in Längsrichtung des Kerns ausgerichtet und mit seiner Nebenachse in Querrichtung des Kerns, entsprechend der bevorzugten Ausrichtung der Fasern im Kern.

Der Superabsorber kann überall im Zentralbereich des Kerns dieselbe Konzentration haben. Ferner kann der Superabsorber im Zentralbereich eine vom Zentrum nach außen allmählich abnehmende Konzentration haben. Der Superabsorber kann ausschließlich im Zentralbereich des Kerns angeordnet sein. Ferner kann in den Seitenbereichen des Kerns der Superabsorber eine geringere Konzentration als im Zentralbereich haben. Der Superabsorber kann in den Seitenbereichen überall die gleiche oder eine nach außen allmählich abnehmende Konzentration haben.

Gemäß einer bevorzugten Ausgestaltung nimmt die Konzentration des Superabsorbers vom Zentrum des Kerns ausgehend zu den beiden Querseiten des Kerns und/oder zu den beiden Längsseiten des Kerns hin ab. Dies entspricht dem Konzentrationsgefälle der Flüssigkeit, die sich ausgehend von der Aufgabenstelle radial nach außen ausbreitet. Ferner ist dieser Kern in der Herstellung nach dem erfindungsgemäßen Verfahren bzw. mit der erfindungsgemäßen Vorrichtung vorteilhaft.

Gemäß einer bevorzugten Ausgestaltung ist der teilchenförmige Superabsorber auf den Kern aufgestreut. Grundsätzlich ist es aber auch möglich, den Superabsorber über die Tiefe des Kerns zu verteilen.

Gemäß einer weiteren Ausgestaltung ist der Superabsorber mittels eines Klebemittels am Kern fixiert. Hierdurch wird verhindert, dass der SAP bezüglich des Kerns wandert bzw. herausfällt. Das Klebemittel ist bevorzugt ein Heißkleber ("Hotmelt").

Gemäß einer weiteren Ausgestaltung hat der Kern einen zentralen Streifenbereich und mindestens einen von einer Längsseite des zentralen Streifenbereichs über den zentralen Streifenbereich gefalteten seitlichen Flügelbereich und ist der Superabsorber zwischen dem zentralen Streifenbereich und dem Flügelbereich angeordnet. Bevorzugt hat der Kern auf beiden Seiten einen seitlichen Flügelbereich, der über den zentralen Streifenbereich gefaltet ist. Der Superabsorber wird zwischen dem zentralen Streifenbereich und dem mindestens einen seitlichen Flügelbereich ein Herausfallen aus dem Kern gesichert.

Die Bindenprodukte können allein aus dem Kern bestehen oder auf der Unterseite des Kerns eine flüssigkeitsundurchlässige Schicht haben. Die flüssigkeitsundurchlässige Schicht verhindert ein Durchsickern der Flüssigkeit zur Wäsche der Verwenderin. Die flüssigkeitsundurchlässige Schicht kann vollständig flüssigkeitsundurchlässig oder nur teilweise flüssigkeitsundurchlässig sein, sodass sie den Flüssigkeitsdurchgang nur einschränkt. Die flüssigkeitsundurchlässige Schicht kann luftdurchlässig sein, damit das Hygieneprodukt "atmen" kann. Die flüssigkeitsundurchlässige Schicht kann eine zusätzliche Materialschicht sein. Hierbei kann es sich beispielsweise um eine Folie oder um textiles Material handeln. Sie kann insbesondere eine hydrophobe textile Materialschicht sein. Ferner kann als flüssigkeitsundurchlässige Schicht eine Klebstoffschicht an der Unterseite des Kerns zum Einsatz kommen. Bei der flüssigkeitsundurchlässigen Schicht kann es sich auch um eine in den Kern integrierte Schicht handeln. Hierbei kann es sich beispielsweise um hydrophobe Fasern handeln, die in die Unterseite des Kerns integriert sind.

Gemäß einer weiteren Ausgestaltung ist auf der Oberseite des Kerns mindestens eine Flüssigkeitsempfangsschicht ("Acquisition layer"), manchmal auch Flüssigkeitverteilschicht ("Distribution layer") genannt, angeordnet.

Diese hat die Funktion, die vom Oberflächenmaterial (Topsheet) aufgenommene Flüssigkeit abzuleiten und aufzunehmen, sie zu verteilen, sie zu "puffern" (zwischenspeichern), um sie dann an den Kern abzuleiten. Der Kern - und insbesondere der Superabsorber - kann Flüssigkeit - besonders bei Schwallbelastungcn - nur verzögert aufnehmen.

Hierbei kann es sich um eine zusätzlich auf der Oberseite des Kerns angeordnete zusätzliche Materialschicht oder um eine in den Kern integrierte Schicht handeln.

Gemäß einer weiteren Ausgestaltung ist auf der Oberseite des Kerns eine flüssigkeitsdurchlässige Deckschicht ("Topsheet") mit textilem Charakter angeordnet. Die Deckschicht dient dazu, einen angenehmen Körperkontakt herzustellen. Hierbei kann es sich um eine zusätzliche Schicht oder um eine in die Außenseite des Kerns integrierte Schicht handeln. Die Deckschicht kann insbesondere eine zusätzliche Schicht aus Nonwoven oder eine perforierte Folie sein. Sie kann zugleich die Flüssigkeitsaufnahme- und/oder Flüssigkeitsverteilschicht sein.

Die nachfolgend beschriebenen Verfahren sind nicht Gegenstand des Patents.

Sie dienen nur dem besseren Verständnis der Erfindung.

Bei dem Verfahren zum Herstellen eines Hygieneproduktes werden
- auf die Oberseite einer Materialbahn aus einem Airlaid, Vlies oder einem anderen flexiblen Material aus natürlichen und/oder künstlichen Fasern lose Teilchen eines Superabsorbers aufgebracht,
- an die Unterseite der Materialbahn in voneinander beabstandeten Bereichen ein Unterdruck angelegt, sodass sich der aufgebrachte Superabsorber auf die Bereiche konzentriert, an die ein Unterdruck angelegt wird,
- bei dem die Materialbahn auf einem Transportband mit einem Lochmuster unterhalb der Bereiche der Materialbahn, in denen der Superabsorber aufkonzentriert werden soll, über eine Einrichtung zum flächigen Anlegen eines Vakuums geführt wird, und der Superabsorber durch Anlegen eines Vakuums an die Unterseite des Transportbandes in den Bereichen der Materialbahn oberhalb der Lochmuster konzentriert wird und
- die Materialbahn zwischen den Bereichen, auf die der Superabsorber konzentriert ist, in streifenförmige, flüssigkeitsabsorbierende Kerne zertrennt.

Bei dem Verfahren wird durch gezieltes Anlegen eines Unterdruckes in voneinander beabstandeten Bereichen der Materialbahn erreicht, das sich der Supcrabsorber auf diese Bereiche konzentriert. Diese Bereiche bilden die Zentralbereiche des Kerns. Die Konzentration des Superabsorbers und die Verteilung des Superabsorbers hängen insbesondere von der Ausbildung der Bereiche ab, an die ein Unterdruck angelegt wird, und von der Höhe des applizierten Unterdruckes. Zwischen den Bereichen, in denen der Superabsorber aufkonzentriert ist, wird die Materialbahn in flüssigkeitsabsorbierende Kerne zertrennt.

Gemäß einer Ausgestaltung wird auf die Oberseite der Materialbahn ein Klebstoff zum Fixieren des Superabsorbers auf der Materialbahn aufgebracht. Der Klebstoff wird bevorzugt vor dem Aufbringen des Superabsorbers auf die Materialbahn aufgebracht. Grundsätzlich kann aber auch nach dem Aufbringen des Superabsorbers und ggfs. nach dem Anlegen des Unterdruckes aufgebracht werden.

Gemäß einer weiteren Ausgestaltung wird mindestens ein seitlicher Bahnbereich der Materialbahn nach dem Aufbringen und Konzentrieren des Superabsorbers auf die Zentralbereiche über die Oberseite des zentralen Bahnbereiches der Materialbahn gefaltet. Hierdurch wird der Superabsorber im Kern gesichert.

Die Materialbahn weist auf einem Transportband mit einem Lochmuster unterhalb der Bereiche der Materialbahn, in denen der Superabsorber aufkonzentriert werden soll, über eine Einrichtung zum flächigen Anlegen eines Vakuums geführt, und der Superabsorber durch Anlegen eines Vakuums an die Unterseite des Transportbandes in den Bereichen der Materialbahn oberhalb der Lochmuster konzentriert. Das Transportband mit dem Lochmuster bildet eine Maske, über die der Unterdruck gezielt auf die Bereiche der Materialbahn gerichtet wird, in denen der SAP konzentriert werden soll.

Gemäß einer weiteren Ausgestaltung wird die Materialbahn oder der Kern mit einer flüssigkeitsundurchlässigen Schicht an der Unterseite und/oder mindestens einer Flüssigkcitsempfangs- und/oder Flüssigkeitsverteilschicht an der Oberseite und/oder an der Oberseite außen mit einer flüssigkeitsdurchlässigen textilen Deckschicht versehen. Diese weiteren Schichten können ganz oder teilweise auf die Materialbahn vor dem Zerteilen oder nach dem Zerteilen der Materialbahn auf den Kern aufgebracht werden. Bevorzugt werden Sie nach dem Zerteilen auf den Kern aufgebracht.

Die Aufgabe wird durch eine Vorrichtung zum Herstellen eines Hygieneproduktes mit den Merkmalen von Anspruch 1 gelöst. Vorteilhafte Ausgestaltungen der Vorrichtung sind in Unteransprüchen angegeben.

Die erfindungsgemäße Vorrichtung zum Herstellen eines Hygieneproduktes hat
- einen Vakuumtisch,
- ein über Umlenkungen geführtes Transportband mit mehreren voneinander beabstandeten Lochmustern, das mit einem oberen Transportbandabschnitt über die Oberseite des Vakuumtisches geführt ist,
- Mittel zum Zuführen einer Materialbahn aus einem Airlaid, Vlies oder aus einem anderen flexiblen Material aus natürlichen und/oder künstlichen Fasern in Laufrichtung des Transportbandes auf die Oberseite des oberen Transportabschnittes,
- Mittel zum Aufgeben von Teilchen eines Superabsorbers auf die Oberseite der Materialbahn auf dem oberen Transportbandabschnitt,
- Mittel zum Anlegen eines Unterdruckes an den Vakuumtisch und
- bei der beidseitig des Transportbandes Leitbleche zum Umlenken von seitlichen Bahnbereichen der Materialbahn auf die Oberseite eines zentralen Bahnbereiches der Materialbahn vorhanden sind.

Bei der erfindungsgemäßen Vorrichtung wird das Vakuum gezielt über das Transportband mit voneinander beabstandeten Lochmustern an Bereiche der Materialbahn angelegt. Hierfür wird die Materialbahn in Laufrichtung des Transportbandes auf die obere Seite des oberen Transpoitbandabschnittes aufgebracht. Auf die Oberseite der auf dem Transportband angeordneten Materialband wird der Superabsorber mittels der Mittel zum Aufgeben aufgebracht. Das Vakuum wird an die Unterseite des oberen Transportbandabschnittes mittels eines Vakuumtisches angelegt. Der Vakuumstisch ermöglicht ein Anlegen eines gleichmäßigen Unterdruckes an eine bestimmte Fläche. Vakuumtische sind dem Fachmann bekannt und kommerziell erhältlich. An dem Vakuumtisch kann mittels einer Vakuumpumpe ein Unterdruck angelegt werden.

Gemäß einer Ausgestaltung wird die Materialbahn von einer Rolle oder einem Block abgezogen und den Mitteln zum Zuführen zugeführt. In einem Block kann die Materialbahn in Zickzack-Faltung abgelegt sein.

Gemäß einer weiteren Ausgestaltung umfassen die Mittel zum Ausbringen eines Supcrabsorbers eine Rutsche für Partikel des Superabsorbers. Gemäß einer weiteren Ausgestaltung ist der Rutsche eine Dosiervorrichtung zum Dosieren des Materialstroms der Teilchen des Superabsorbers vorgeordnet.

Ferner sind gemäß einer Ausgestaltung Mittel zum Aufgeben eines Klebstoffes auf die Oberseite der Materialbahn vorhanden. Gemäß einer weiteren Ausgestaltung sind die Mittel zum Aufgeben eines Klebstoffes in Durchlaufrichtung der Materialbahn vor den Mitteln zum Aufgeben eines Superabsorbers angeordnet. Grundsätzlich können die Mittel zum Aufgeben eines Klebstoffes aber auch in Durchlaufrichtung hinter dem Mittel zum Aufbringen eines SAP oder hinter dem Vakuumtisch angeordnetsein.

Gemäß einer weizeren Ausgestaltung umfassen die Mittel zum Aufgeben eines Klebstoffes einen Sprühkopf für einen Klebstoff. Vorzugsweise handelt es sich hierbci um einen Sprühkopf für einen Heißklebstoff.

Beidseitig des Transportbandes sind Leitbleche zum Umlenken von seitlichen Bahnbereichen der Materialbahn auf die Oberseite eines zentralen Bahnbereichs der Materialbahnvorhanden. Die Mittel zum Umlenken schlagen die seitlichen Bahnbereiche über den zentralen Bahnbereich und sichern hierdurch den SAP der Materialbahn.

Gemäß einer weiteren Ausgestaltung sind in Laufrichtung hinter dem Transportband Mittel zum Zertrennen der Materialbahn in streifenförmige Stücke vorhanden.

Gemäß einer weiteren Ausgestaltung weist die Vorrichtung Mittel zum Synchronisieren der Mittel zum Zertrennen mit der Bewegung des Transportbandes auf. Die Mittel zum Synchronisieren stellen sicher, dass die Materialbahn zwischen den Bereichen zertrennt wird, in denen der Superabsorber konzentriert ist.

Die Erfindung wird nachfolgend anhand der anliegenden Zeichnungen eines Hygieneproduktes und einer erfindungsgemäßen Vorrichtung zur Herstellung des Hygieneproduktes näher erläutert. In den Zeichnungen zeigen:
- Fig. 1: eine Binde mit teilweise ausgebrochener Deckschicht in einer Perspektivansicht schräg von oben und von der Seite;
- Fig. 2 a + b: dieselbe Binde in einem Querschnitt mit ausgestreckten Flügeln (Fig. 2a) und mit zusammengefalteten Flügeln (Fig. 2b);
- Fig. 3: dieselbe Binde in einem Längsschnitt;
- Fig. 4: eine Vorrichtung zum Herstellen eines Hygieneproduktes in einem vertikalen Längsschnitt;
- Fig. 5: einen Abschnitt eines Transportbandes mit Vorrichtungen einer vergrößerten Draufsicht;
- Fig. 6a-c: Binden mit zentral und an den Längsseiten angeordnetem oder umlaufendem Superabsorber jeweils in der Draufsicht.

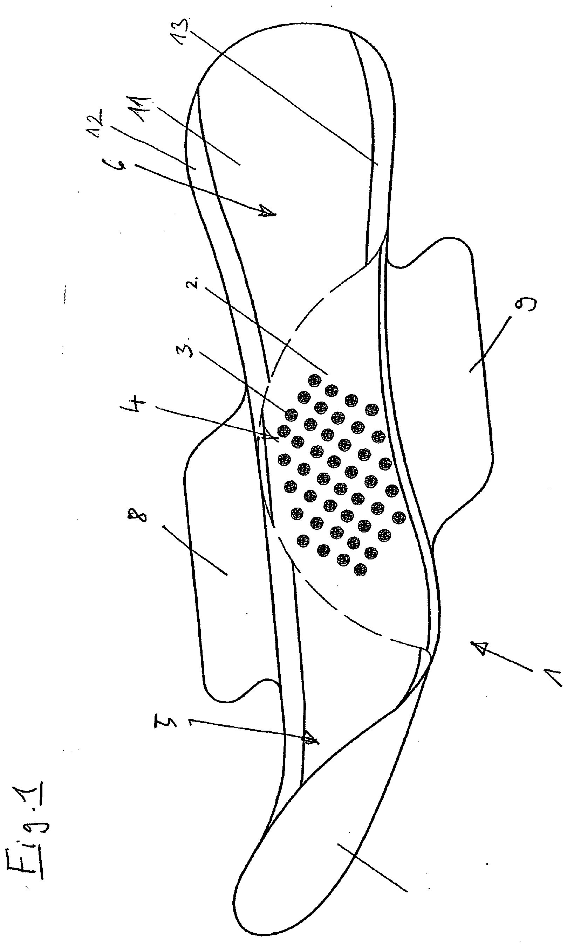

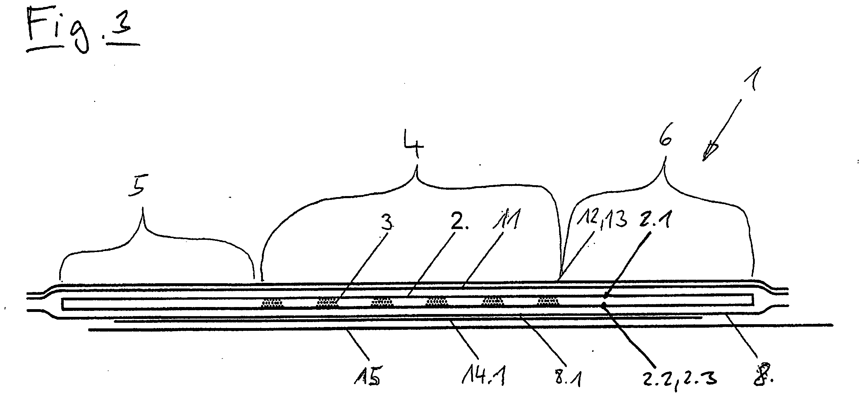

In Fig. 1 bis 3 ist eine Binde 1 gezeigt. Hierbei handelt es sich um eine "Ultrabinde", d.h. um eine besonders dünne Damenbinde, die einen besonders hohen Tragekomfort bietet.

Die Binde 1 weist einen streifenförmigen Kern 2 auf, der aus einem Airlaid gebildet ist. Der Kern 2 hat einen zentralen Streifenbereich 2.1 und zwei von den Längsseiten des zentralen Streifenbereichs 2.1. gefaltete Flügelbereich über diesen gefaltete seitliche Flügelbereiche 2.2, 2.3, die einander auf der Längsmittelachse des Kernes 2 etwas überlappen.

Zwischen dem zentralen Streifenbereich 2.1 und den seitlichen Flügelbereichen 2.2, 2.3 ist ein Superabsorber 3 angeordnet. Hierbei handelt es sich um ein grobkörniges Pulver. Wie am besten in Fig. 1 zu sehen ist, ist der Superabsorber 3 auf einen Zentralbereich 4 des Kerns konzentriert. Dort nimmt er eine annähernd prismenförmige Fläche ein. Innerhalb dieses Zentralbereichs 4 ist der Supcrabsorber 3 in mehreren kleinen, kreisförmigen Häufchen angesammelt. Diese beruhen darauf, dass die Verteilung des Superabsorbers 3 über den Kern mittels einer Lochmaske mit einem der Anordnung der Häufchen entsprechenden Loch Muster gesteuert wird, an das ein Unterdruck angelegt wird.

Der Superabsorber 3 ist mittels Heißklebstoff im Kern 2 fixiert.

In den beiden in Längsrichtung des Kerns 2 beidseitig des Zentralbereiches 4 angeordneten Seitenbereichen 5, 6 des Kerns 2 ist kein oder im Wesentlichen kein Superabsorber 3 vorhanden.

Auf der Unterseite 2 ist der Kern von einem Zentralabschnitt 8.1 einer flüssigkeitsundurchlässigen Schicht ("Wäscheschutzschicht") 8 abgedeckt. Die flüssigkeitsundurchlässige Schicht 8 ist beidseitig neben dem Zentralbereich 4 des Kerns 2 zu zwei nach auben vorstehenden Plügelabschnitten 8.2, 8.3 erweitert, die untere Schichten von Flügeln 9, 10 sind.

Die Oberseite des Kerns 2 ist von einer flüssigkeitsdurchlässigen Deckschicht 11 aus Vlies (auch "Nonwoven" genannt) abgedeckt. Die Deckschicht 11 kann zugleich eine Flüssigkeitsempfangsschicht bilden.

Die beiden längsseitigen Ränder der Deckschicht 11 sind jeweils von den Randseiten von einer seitlichen Deckschicht 12, 13 überdeckt. Die seitlichen Deckschichten 12, 13 bilden die Oberseiten der Flügel 8, 9. Hierbei kann es sich um Schichten mit textilem Charakter (z.B. aus Nonwoven) handeln, die verhindern, dass der Körper in Kontakt mit der Kunststofffolie kommt, die die flüssigkeitsundurchlässige Schicht 8 und die unteren Schichten der Flügel 8, 9 bilden kann.

Die verschiedenen Lagen der Binde 1 können an geeigneten Stellen durch Klebstoff miteinander verbunden werden. Insbesondere kann Heißklebstoff zum Einsatz kommen.

Bevorzugt ist der Zentralabschnitt 8.1 der flüssigkeitsundurchlässigen Schicht 8 mit der Unterseite des Kerns 2 verklebt. Weiterhin bevorzugt ist die flüssigkeitsdurchlässige Deckschicht 11 punktuell mit der Oberseite des Kerns 2 verklebt, um den Flüssigkeitsdurchgang nicht zu behindern. Ferner sind die weiteren Deckschichten 12, 13 mit den Flügelabschnitten 8.2, 8.3 der flüssigkeitsundurchlässigen Schicht 8 und den Randbereichen der Deckschicht 4 verklebt.

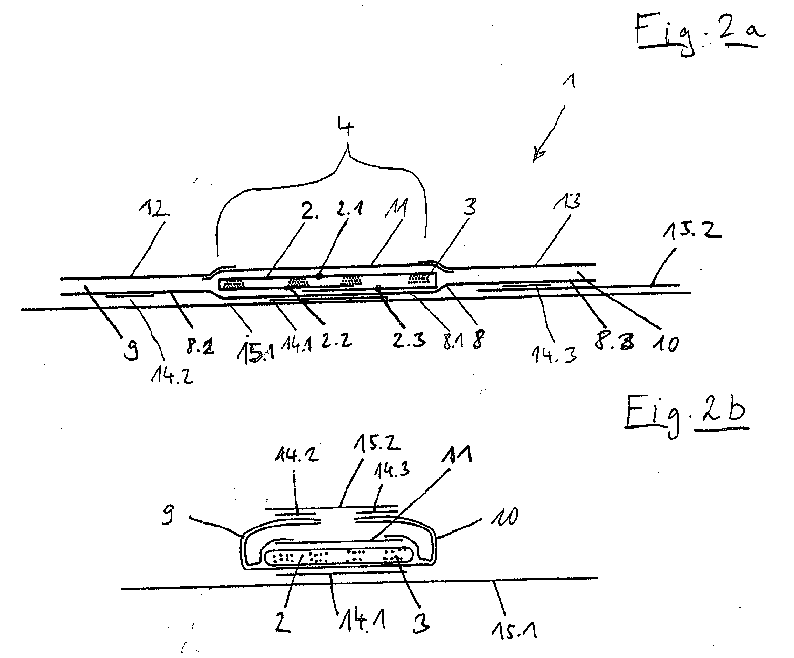

An der Unterseite der flüssigkeitsundurchlässigen Schicht 8 sind Haftleimstreifen 14.1, 14.2, 14.3 zum Fixieren am Steg eines Höschens vorhanden. Der Haftleimstreifen 14.1 ist von einem mittig aufliegendem Abdeckmaterial 15.1 abgedeckt. Ferner ist der Haftleimstreifen 14.3 von einem darauf aufliegendem, streifenförmigen, weiteren Abdeckmaterial 15.2 abgedeckt, der in Fig. 2a seitlich vorsteht. Die Abdeckmaterialien 15.1, 15.2 sind beispielsweise aus einem silikonisierten Papier oder aus einer silikonisierten Folie. Die Silikonisierung erleichtert das Abziehen des Abdeckmaterials 15.1, 15.2.

Gemäß Fig. 2b sind die Flügel 9, 10 über die flüssigkeitsdurchlässige Deckschicht 11 geschlagen und decken das Abdeckmaterial 15.2 die Haftleimstreifen 14.2 und 14.3 ab. Das Abdeckmaterial 15.1 dient zugleich als Einzelverpackungsfolie bzw. als äußere Verpackungshülle der Binde 1.

Die Binde 1 wird mittels der Flügel 8, 9 am Steg eines Höschens fixiert, sodass abgegebene Körperflüssigkeit zentral über dem Zentralbereich 4 des Kerns 2 abgegeben wird. Von dort wird sie durch die Deckschicht 11 und den Kern 2 radial nach außen verteilt, sodass sie beim Erreichen der längsseitigen Ränder des Kerns 2 beinahe den gesamten Zentralbereich 4 einnimmt und von dem dort angeordneten Superabsorber 3 gebunden wird. Hierdurch wird bei geringstmöglichem Einsatz von SAP 3 die Kapazität der Binde 1 optimal ausgenutzt.

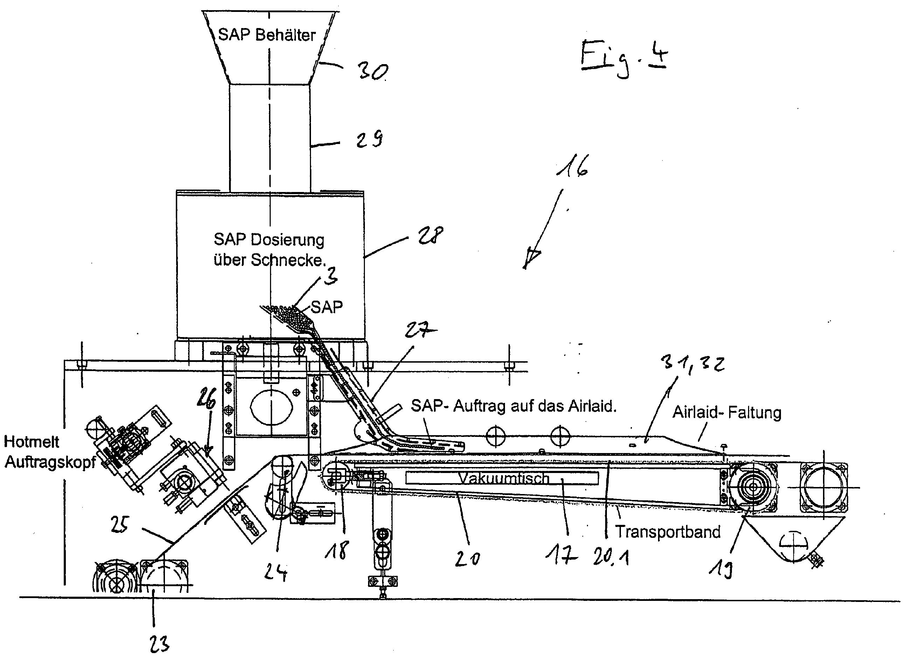

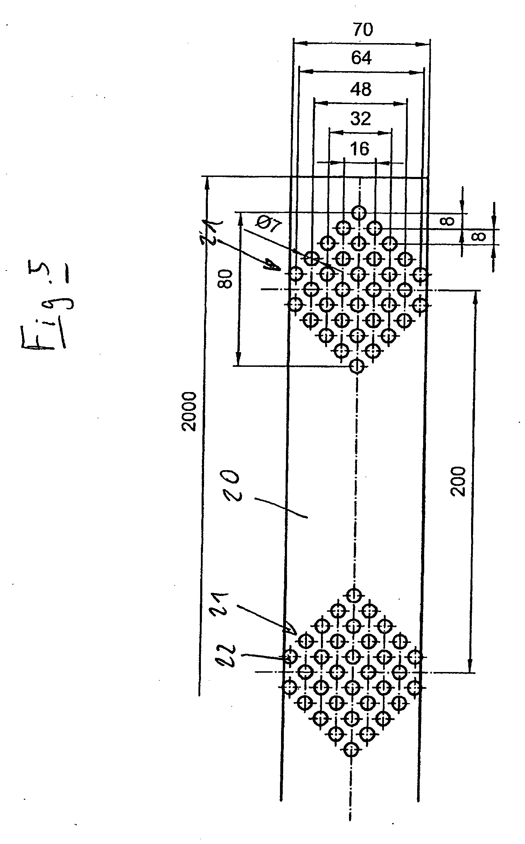

Gemäß Fig. 4 und 5 weist eine Vorrichtung 16 zum Herstellen des Kerns 2 einen Vakuumtisch 17 auf. Um den Vakuumtisch 17 herum ist über Umlenkrollen 18, 19 ein Transportband 20 geführt. Der obere Transportbandabschnitt 20.1 des Transportbandes 20 verläuft direkt über den Vakuumtisch 17. Das Transportband weist Lochmuster 21 auf, mit jeweils einer Vielzahl auf einer prismenförmigen Fläche angeordneten Löchern 22, die in Fig. 5 gezeigt sind. Außerhalb der Lochmuster ist das Transportband geschlossen.

Eine der Umlenkrollen 19 des Transportbandes 20 ist von einem Motor angetrieben.

Über weitere Rollen 23, 24 wird der Oberseite des oberen Transportbandabschnittes 20.1 des Transportbandes 20 eine Materialbahn 25 aus Airlaid zugeführt.

Vor dem Transportband 20 ist oberhalb der Materialbahn 25 ein Sprühkopf 26 für einen Heißklebstoff angeordnet.

Oberhalb des oberen Transportbandabschnittes 20.1 des Transportbandes 20 ist eine Rutsche 27 für pulverförmiges SAP angeordnet. Die Rutsche 27 wird von einer Dosiereinrichtung 28 mit einer Dosierschnecke gespeist. Die Dosierschnecke wird aus einem Vorratsbehälter 29 für SAP mit einem Aufgabetrichter 30 gespeist.

Beidseitig des oberen Transportbandabschnittes 20.1 des Transportbandes 20 sind Führungsschienen 31, 32 zum Falten seitlicher Bahnbereiche der Materialbahn 25 über einen zentralen Bahnbereich der Materialbahn 25 angeordnet.

Diese Vorrichtung funktioniert wie folgt:

Die Materialbahn 25 aus Airlaid wird als Rollenware oder im Block zickzackförmig bzw. "festoont" abgelegt geliefert und auf die Vorrichtung 16 aufgelegt. Die Materialbahn 25 wird so aufgelegt, dass sie über die Rollen 23, 24 auf dem oberen Transportbandabschnittes 20.1 des Transportbandes 20 geführt wird. Der Vakuumtisch 17 unterhalb des oberen Transportbandabschnittes 20.1 zieht Luft durch die Lochmuster 21, wodurch die auf dem oberen Transportbandabschnitt 20.1 liegende Materialbahn 25 fixiert wird. Die seitlichen Führungen 31, 32 stellen die Materialbahn 25 im Bereich des oberen Transportbandabschnittes 20.1 zunächst U-förmig auf.

Über die Dosiereinrichtung 28 wird der Rutsche 27 eine gleichmäßige Menge SAP in Gramm pro Zeiteinheit zugeführt. Die zugeführte Menge kann in Abhängigkeit von der Bandgeschwindigkeit des Transportbandes 20 variieren. Über die Rutsche 27 wird das eindosierte SAP auf die Oberseite der U-förmig aufgestellten Materialbahn 25 gegeben.

Über den Sprühkopf 26 wird auf die Materialbahn 25 vor dem Auflaufen auf das Transportband 20 Heißklebstoff aufgesprüht.

Die SAP-Körnchen gelangen von der Rutsche 27 auf die Materialbahn 25, werden dabei entsprechend beschleunigt und verteilen sich auf der Materialbahn 25. Aufgrund der Lochmuster 21 in dem Transportband 20, an die über den Vakuumtisch 17 ein Unterdruck angelegt wird, legt sich der SAP bevorzugt über den Lochmustern 21 ab. Das Vakuum erzeugt nämlich einen Luftstrom, der von oben durch das Airlaid hindurch zum Vakuumtisch 17 gerichtet ist und die SAP-Körnchen zu den Stellen oberhalb der Lochmuster 21 transportiert.

Der SAP legt sich bevorzugt entsprechend der Löchergeometrie des Transportbandes 20 auf der Materialbahn 25 ab und wird durch den Heißklebstoff auf der Materialbahn 25 fixiert, wenn er sich nicht mehr relativ zur Materialbahn 25 bewegt. Zusätzlich wird der SAP durch die seitlichen Bahnbereiche der Materialbahn 25 fixiert, die von den Führungen 31, 32 in Laufrichtung hinter dem Vakuumtisch 17 über den zentralen Bahnbereich gefaltet werden.

Das Layout der Lochmuster 21 und das Vakuum können so gesteuert werden, dass ein bestimmter Anteil des SAP auf der Materialbahn 25 verbleibt und gleichmäßig über die Länge der Materialbahn 25 verteilt wird.

Die Materialbahn 25 wird schließlich so synchron in Kerne 2 geschnitten, dass der Bereich mit höchster Konzentration an SAP 3 mittig im Kern angeordnet ist.

Die erfindungsgemäße Vorrichtung 16 kann mit nur geringfügigen Modifikationen an bestehenden Vorrichtungen zur Herstellung von Kernen 2 mit SAP-Dosierung verwirklicht werden.

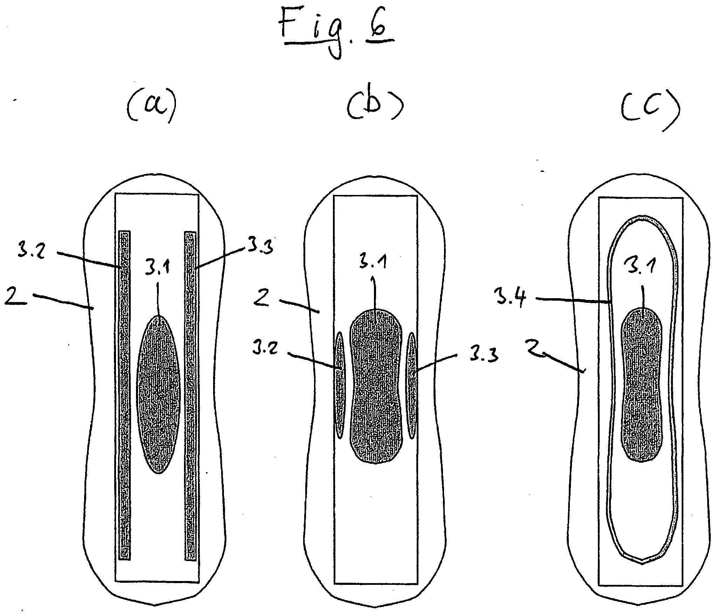

Gemäß Fig. 6a-c ist SAP 3.1 jeweils mittig in einem Zentralbereich des Kerns 2 angeordnet. Darüber hinaus gibt es bei Fig. 6a und 6b Seitenbereiche 3.2, 3.3 aus SAP und bei Fig. 6c einen um den Zentralbereich 3.1 umlaufenden Streifenbereich 3.4 aus SAP. Die Seitenbereiche 3.2, 3.3, 3.4 dienen als Seitenauslaufschutz.

## Patentansprüche

1. Vorrichtung zum Herstellen eines Hygieneproduktes mit
- einem Vakuumtisch (17),
- einem über Umlenkungen (18, 19) geführten Transportband (20) mit mehreren voneinander beabstandeten Lochmustern (21), das mit einem oberen Transportbandabschnitt (20.1) über die Oberseite des Vakuumtisches (17) geführt ist,
- Mitteln zum Zuführen (23, 24) einer Materialbahn (25) aus einem Airlaid, Vlies oder einem anderen flexiblen Material aus natürlichen und/oder künstlichen Fasern in Laufrichtung des Transportbandes (20) auf die Oberseite des oberen Transportbandabschnittes (20.1),
- Mitteln zum Aufgeben eines Superabsorbers (27) auf die Oberseite der Materialbahn (25) auf dem oberen Transportbandabschnitt (20.1) des Transportbandes (20),
- Mitteln zum Anlegen eines Vakuums an den Vakuumtisch (17) und
- bei der beidseitig des Transportbandes Leitbleche (31, 32) zum Umlenken von seitlichen Bahnbereichen der Materialbahn (25) auf die Oberseite eines zentralen Bahnbereiches der Materialbahn (25) vorhanden sind.

2. Vorrichtung nach Anspruch 1, bei der die Mittel zum Aufbringen eines Superabsorbers eine Rutsche (17) für den Superabsorber (3) umfassen und der Rutsche (17) eine Dosiervorrichtung (28) zum Dosieren des Materialstroms des Superabsorbers (3) vorgeordnet ist.

3. Vorrichtung nach Anspruch 1 oder 2, bei der die Mittel zum Aufgeben eines Klebstoffes (26) in Durchlaufrichtung der Materialbahn (25) vor den Mitteln zum Aufgeben eines Superabsorbers (27) angeordnet sind.

4. Vorrichtung nach einem der Ansprüche 1 bis 3, bei der in Laufrichtung hinter dem Transportband (20) Mittel zum Zerteilen der Materialbahn (25) in streifenförmige Kerne (2) vorhanden sind und die Mittel zum Synchronisieren der Mittel zum Zerteilen mit der Bewegung des Transportbandes (20) hat.

## Claims

1. A device for producing a hygiene product, comprising
- a vacuum table (17),
- a transportation belt (20) which is guided over deflectors (18, 19), which has multiple mutually spaced hole patterns (21), and which is guided by an upper transportation belt portion (20.1) over the upper face of the vacuum table (17),
- means for supplying (23, 24) a material web (25) consisting of an air-laid, nonwoven, or other flexible material consisting of natural and/or synthetic fibers, in the travel direction of the transportation belt (20) onto the upper face of the upper transportation belt portion (20.1),
- means for applying a super-absorber (27) onto the upper face of the material web (25) on the upper transportation belt portion (20.1) of the transportation belt (20),
- means for applying a vacuum to the vacuum table (17), and
- wherein guide plates (31, 32) for deflecting lateral web regions of the material web (25) onto the upper face of a central web region of the material web (25) are provided on both sides of the transportation belt.

2. The device according to claim 1, wherein the means for applying a super-absorber comprise a chute (17) for the super-absorber (3) and a metering device (28) for metering the material flow of the super-absorber (3) is arranged upstream of the chute (17).

3. The device according to claim 1 or 2, wherein the means for applying an adhesive (26) are arranged upstream of the means for applying a super-absorber (27) in the throughput direction of the material web (25).

4. The device according to one of claims 1 to 3, wherein means for cutting the material web (25) into strip-shaped cores (2) are provided downstream of the transportation belt (20) in the travel direction and has the means for synchronizing the cutting means with the movement of the transportation belt (20).

## Revendications

1. Dispositif de fabrication d'un produit hygiénique comprenant
- une table aspirante (17),
- un tapis roulant (20) guidé par le biais de déflecteurs (18, 19) avec plusieurs motifs à trou (21) espacés les uns des autres, lequel est guidé avec une partie supérieure de tapis roulant (20.1) au-dessus de la face supérieure de la table aspirante (17),
- des moyens (23, 24) destinés à conduire une nappe de matériau (25) constituée d'un airlaid, d'un non-tissé ou d'un autre matériau flexible en fibres naturelles et/ou synthétiques sur la face supérieure de la partie supérieure de tapis roulant (20.1) dans la direction de marche du tapis roulant (20),
- des moyens destinés à appliquer un super-absorbant (27) sur la face supérieure de la nappe de matériau (25) sur la partie supérieure de tapis roulant (20.1) du tapis roulant (20)
- des moyens destinés à appliquer un vide à la table aspirante (17) et
- dans lequel il est prévu des tôles de guidage (31, 32) de part et d'autre du tapis roulant pour la déviation de zones de nappe latérales de la nappe de matériau (25) vers la face supérieure d'une zone de nappe centrale de la nappe de matériau (25).

2. Dispositif selon la revendication 1, dans lequel les moyens destinés à appliquer un super-absorbant comportent une glissière (17) pour le super-absorbant (3) et un dispositif de dosage (28) destiné au dosage du flux de matériau du super-absorbant (3) est placé en amont de la glissière (17).

3. Dispositif selon la revendication 1 ou 2, dans lequel les moyens destinés à appliquer un adhésif (26) sont arrangés en amont des moyens destinés à appliquer un super-absorbant (27) dans la direction de marche de la nappe de matériau (25).

4. Dispositif selon l'une des revendications 1 à 3, dans lequel il est prévu des moyens destinés à découper la nappe de matériau (25) en noyaux en forme de bande (2) en aval du tapis roulant (20) dans la direction de marche et lequel possède des moyens destinés à synchroniser les moyens destinés à découper avec le déplacement du tapis roulant (20).
